# EUROPEAN PATENT APPLICATION

(11) **EP 1 338 307 A1**
(43) Date of publication of application: **27.08.2003**
(21) Application number: 03250906.9
(22) Date of filing: 14.02.2003
(51) Int. Cl.: A62D 3/00

(54) **Chemical and biological decontamination material and system**

(30) Priority: 22.02.2002 US 358838 P
(71) Applicant: ROHM AND HAAS COMPANY, Philadelphia, Pennsylvania 19106-2399 (US)
(72) Inventor: Rosenbaum, Bruce Maurice, Fort Washington, Pennsylvania 19034 (US)
(74) Representative: Kent, Venetia Katherine

(57) **Abstract**

Methods for biological and chemical decontamination comprising contacting said biological and chemical toxins with a carbonaceous, polymeric or mineral adsorbent in which elemental or ionic silver is contained in the pores of said adsorbent.

## Description

### Background of the Invention

With the advent of chemical warfare agents such as mustard gases discovered in the early 1800's and used in WWI and nerve agents,(discovered in the mid-1930's and used by terrorists in Japan in the 1990's, effective methods for their decontamination have been pursued. While biological warfare agents have been used even longer than chemical agents, for example via the willful dispersal of materials containing disease pathogens to susceptible populations (e.g, smallpox), and more recently anthrax dispersed via the US mail in late 2001 and2002, effective methods of protection against and decontamination of biological warfare agents have been elusive.

Chemical agents such as VX, GD, and Sarin (GB) (organophosphorous nerve agents), and H or HD ( organosulfur or nitrogen mustard blister agents) are known to be inactivated by either adsorption or chemical decomposition.

Various methods of contacting chemical agents with strong caustic (lye) have been adopted and used in the past. The major drawback is that caustic is itself toxic and corrosive, especially to skin. Other formulations (eg. DS2 used by the US Army, C8-emulsion used by NATO) contain, in addition to caustic, DETA, EGME, and / or hydrogen peroxide, or calcium hypochlorite (chlorine bleach), perchloroethylene (or carbon tetrachloride). These ingredients also pose potential health risks.

Attempts to formulate less dangerous mixtures have resulted in combinations of sodium or potassium bicarbonate, solid urea or aqueous hydrogen peroxide, and alcohol. See ,US6245957 . While this mixture may be effective for cleaning equipment contaminated with chemical agents, it may be too slow acting to decontaminate personnel, and is, in any case, ineffective in the decontamination of biological agents.

Another formulation ,as disclosed in US5859064 ,is a solution made of about 20% of a quarternary ammonium complex (containing benzyltrimethylammoniumchloride and benzyltriethylammonium chloride) and about 20% oxidizer (hydrogen peroxide or perborates or peracetates or peroxyphthalates or peroxysulfates or percarbonates) in water or glycol. While effective against GD (99% destruction in 30 seconds), it is slower acting against VX (37% destruction in 30 seconds, 99% destruction in one hour), and much slower acting against HD (20% destruction in 30 seconds, 66% destruction in one hour). This too is ineffective against biological agents.

Another formulation is a barrier cream, as disclosed in US5075297, consisting of an alkali metal (eg. potassium) salt of phenol or of an oxime (eg. acetophenone oxime, acetone oxime, 2,3-butanedione monoxime) as its active ingredient, combined with a solvent for the active ingredient, contained within a waxy/ creamy solid macrocycle base (to which inert thickeners can be added). The patent cites effective destruction of HD, VX, and GD agents in 5 minutes in a reaction vessel containing diluted active ingredient, base, and agent (0.2 molar active ingredient). Effective ratio of active ingredient:base:agent was 8:8:1, which quickly dropped as the ratio of active ingredient to agent dropped, or the ratio of active ingredient to base dropped. Also no anti-microbial activity occurs with this cream.

Another approach is the use of aqueous microemulsions containing alkanes, anionic surfactants (eg. sodium lauryl sulfate, ie. soap), and alcohols. See, US5695775. This may work somewhat for chemical agents (although a soapy wet rag may work just as well), but will not be effective in the decontamination of biological agents.

Other approaches have been the use of sorbents to remove chemical agents from skin. Fuller's earth and F-200 activated alumina are two such examples. The problem with this approach is the possibility of subsequent desorption and redeposition onto surfaces or into the air. Ambergard™ XE555, currently used in the US Army M-291 Personnel Skin Decon Kit, similarly uses an adsorbent, but also uses anionic and cationic resins to provide a method of chemical agent destruction. An improvement on this approach is described in US5336329, where a strong base hydroxide functionality has been added to the sorbent. While these embody perhaps the most effective approaches to the decontamination and destruction of chemical agents, they are, unfortunately, ineffective in the decontamination of biological agents.

Biological agents are more difficult to inactivate. Spore-forming bacteria, such as anthrax, can be highly resistant. There exist anti-microbial agents that can kill these species, but few that are compatible with human skin. For example formaldehyde and chlorine dioxide are effective, but toxic. Isothiazalones may be effective, but they are of no value against chemical agents. Iodine, also effective against microbes, can form explosive compounds when dried.

A foam developed for the decontamination of equipment (WO01/56380, US Provisional Appl 60/176499) uses biocides (preferably triclosan, tetrakishydroxymethyl phosphonium sulfate, and benzyalkonium chloride), a chemical binding agent (preferably bovine serum), and an enzyme (preferably organophosphorous acid anhydrase). Unfortunately this is not a viable approach for skin decontamination as it is slow acting (at least one hour), and requires elaborate storage canisters and a foam distribution apparatus

Garments and air filters have been made using fabrics and filter media containing carbon powders, carbon impregnated rubber foams, as well laminates using synthetic carbon adsorbents in bead form . See, US5769992. While effective in the adsorption of chemical agents, there is no anti-microbial efficacy.

Antimicrobial fabrics are described in US5662991 that employ iodinated polypropylene resins, and in fact iodinated synthetic resins are known in the art. However these materials are ineffective against chemical agents.

Thus, there is a need in the art for a means of preventing and treating contamination by both chemical and biological warfare agents. Applicant's invention meets said need.

### Summary of the Invention

The present invention relates to a method for preventing and treating the biological and chemical contamination of a mammalian subject comprising topically administering to said subject a silver impregnated adsorbent.

The present invention further relates to a method for preventing and treating the biological and chemical contamination of a gas stream comprising contacting said gas stream with a silver impregnated adsorbent.

The present invention also relates to a method for preventing biological and chemical contamination of a mammalian subject comprising wearing protective clothing treated with a silver impregnated adsorbent.

### Detailed Description the Invention

The present invention relates to a method for preventing and treating the biological and chemical contamination of a mammalian subject comprising topically administering to said subject a silver impregnated adsorbent.

The present invention further relates to a method for preventing and treating the biological and chemical contamination of a gas stream comprising contacting said gas stream with a silver impregnated adsorbent.

The present invention also relates to a method for preventing biological and chemical contamination of a mammalian subject comprising wearing protective clothing treated with a silver impregnated adsorbent.

Elemental silver and silver salts have been used for many years safely and effectively against microbes. Silver nitrate solution is commonly used as a prophylactic measure against eye infections in newborns. It is also used extensively in catheters. In recent years silver has been loaded onto activated carbon for use in the purification of drinking water, as in various water pitcher products, the silver's purpose to prevent the growth of bacteria on the moist carbon substrate, depleted of chlorine. The present invention uses the anti-microbial activity of elemental and ionic silver together with the adsorptive properties of various porous substrates to offer protection against toxic materials, both chemical and biological. Silver useful in the practice of the present invention is a water soluble silver salt, such as silver nitrate.

The adsorbents useful in the practice of the present invention include, but are not limited to, porous carbonaceous adsorbents, such as Rohm and Haas' Ambersorb™ carbonaceous adsorbent or activated carbon; porous polymeric adsorbents, such as Rohm and Haas' Amberchrome® CG300 polymeric adsorbent; functionalized porous ion exchangers such as Amberlyst XN1010Dry (sulfonated poly-styrene/DVB) and Ambersep 900OH (aminated poly-styrene/DVB) available from the Rohm and Haas Company ,or a porous mineral adsorbent, such as activated alumina, zeolites, or silicas. Elemental or ionic silver is impregnated or imbibed onto said adsorbent.

If desired ,carbonaceous adsorbents may be prepared by the pyrolysis and steam activation of naturally occurring carbon sources, such as bituminous coal or coconut shells, or through the use of synthetic materials, such as styrene/DVB polymers to which carbon fixing moieties, such as sulfate, have been added. See, US4957897.

If desired, porous polymeric adsorbents may be prepared by the suspension polymerization of one or more monomers to which a solvent partially soluble in the water phase and the monomer phase, but insoluble in the polymeric phase is used.

Mineral adsorbents may be naturally occurring, for example various clays (Fuller's Earth, lamellar clays), or prepared by activation with steam, as in the case of activated alumina, or can be made via reverse-phase polymerization, as in the case of silicas and zeolites.

Preferred adsorbents include , but are not limited to Ambersorb™ carbonaceous adsorbent, activated alumina, and Amberchrome® CG300 polymeric adsorbent. More preferred adsorbents include , but are not limited to Ambersorb™ carbonaceous adsorbent and activated alumina.
The most preferred adsorbent is Ambersorb™ carbonaceous adsorbent.

Methods for loading adsorbents with silver are known to those skilled in the art. Generally, loading of the adsorbents with silver can be done by allowing the adsorbent to contact a silver salt solution and then heating to evaporate the water. The degree of elemental silver reduction may be controlled to a certain extent by the pre-treatment of the adsorbent with nitric acid , or by the heating of the silver salt laden adsorbent to temperatures at which reduction occurs, eg. heating a carbonaceous adsorbent laden with silver nitrate to 300C as described in US3294572, or by the subsequent addition and washing with aqueous solutions of ammonia, formaldehyde, caustic, and sodium chloride.

Said silver loaded adsorbents prepared as described hereinabove can be dried and ground to a powder of any desired particle size by means known to those skilled in the art. Finely ground powders are preferred as they are amenable to administering by wiping, sprinkling or spraying. The powders can also be blended into slurries or creams for topical application. The methods of preparation of slurries and creams are known to those skilled in the art. Typical ingredients for the preparation of slurries and creams are water, mineral oil, petrolatum, fatty acids, silicones, glycerin, vitamin E,xanthan gum and fatty alcohols.

In one embodiment of the invention, the silver impregnated adsorbents , prepared as described hereinabove, are administered to contaminated mammalian skin as a powder, and the chemical and biological agent is adsorbed and inactivated. The silver impregnated adsorbents may be mixed with anionic and cationic ion exchange resins for improved performance against chemical agents. The silver impregnated adsorbents may be placed within an open weave pouch which can serve as an application pad as long as its construction allows the powder to flow freely through the weave and contact the contaminated skin surface. Additional methods of delivery include, but are not limited to, wipes and spray powder canisters.

In another embodiment of the invention silver impregnated adsorbents prepared as described hereinabove can be affixed to or contained within in a fabric or filter for use as protective garments or air or mist filtration devices such as respirators or gas masks.

Yet another embodiment of the present invention is its usefulness for surface decontamination. Surfaces include, but are not limited to vehicles, equipment, weapons, gas masks, clothing, furniture , buildings and temporary structures. The silver impregnated adsorbent prepared as described hereinabove is simply applied to the surface. Means of application include, but are not limited to wipes and sprays.

The present invention is useful against chemical agents including but not limited to organo sulphur and mustard blistering agents and organo phosphorous nerve toxins.

The present invention is useful against biological agents including but not limited to bacillus species (active and spores), viruses (eg. Venezuelan Equine Encephalitis and small pox ), T-2 mycotoxins, and fungi.

The following non limiting examples illustrate the preparation and utility of the present invention.

### Example 1

Ambersorb CB carbonaceous adsorbent resin, which contains a soluble form of the antimicrobial agent silver, was evaluated for its static and cidal activity against *B. subtilis* in a series of laboratory tests. This silver-containing resin demonstrated excellent activity against a strain of *Bacillus* subtilis in both slurry and solid form. The Ambersorb CB resin showed inhibition of growth of the bacteria on solid agar media and both rapid and extensive killing in a liquid and hard surface efficacy test.

The silver adsorbed resin was prepared as follows: 10 grams of a carbonaceous adsorbent having a surface area of 750 sq m/g was washed in a column with 100 ml DI water. Then 50 ml of 23% HNO₃ followed by 150 ml DI water was run through the column. The washed resin was then placed in a beaker and stirred with 32.7 grams of 1N AgNO₃ and heated at 80C until just wet. It was then placed in a 110C oven and dried overnight. The spherical beads were then ground to a fine powder for anti-microbial testing.

Ambersorb Carbonaceous Adsorbent with SA=750 m²/g loaded with AgNO₃ contains 19.8% Ag ("Ambersorb CB"), as determined by inductively coupled plasma emission.

Unloaded Ambersorb Carbonaceous Adsorbent with SA=750 m²/ g ("Ambersorb C")

AgNO₃ was used as a control at 31% (which contains 19% Ag)

The tests used Ambersorb CB (with Ag) and Ambersorb C (without Ag) as is (solid powder) and in slurry form. The slurry was made by adding 1 g of the solid in 5 g of sterile DI water. Mixed vigorously to form slurry. Ambersorb CB was not soluble but was dispersable in the water. The solid form of Ambersorb CB contained 19.8% Ag whereas the slurry form contained 3.3% Ag.

AgNO₃ was used at 31% (which contained 19.7% Ag, comparable to solid form of Ambersorb CB) and at 5.2% (which contained 3.3% Ag, comparable to slurry form of Ambersorb CB).

AgNO₃ and Ambersorb C were included in the tests for comparison.

An overnight culture of *Bacillus subtilis* (ATCC # 6461, a simulant for the causative agent of anthrax) in Nutrient Broth (incubated in a shaker water bath at 30°C) was used as the inoculum. The nutrient broth solution contained approximately 4x10⁸ colony forming unit (CFU) per ml.

The following three tests were performed to evaluate Ambersorb CB anti-bacterial activity: Zone of Inhibition, Hard Surface Test, Speed of Kill

**Zone of Inhibition-** This test measures the ability of the biocide to inhibit bacterial growth inhibit (static effect), over a 24 hour period , when placed on an agar surface. The larger the diameter (cm) of the zone, the greater the inhibitory effect.

The test also measures the ability of Ambersorb CB to diffusion into the agar. The greater the diameter of the zone, the greater the inhibitory effect.

### Zone of Inhibition Test

In this test, bacteria are added to molten agar and allowed to solidify. The chemical is added to the surface of the solid agar and the plate is incubated overnight. The zone of inhibition is measured as the diameter where no growth of the bacteria is observed on the surface of the agar. The greater the zone, the greater the extent of inhibition.

To prepare the solid agar media for testing, 0.23 ml of the *Bacillus* inoculum was added to 23 ml of molten nutrient agar in a petri dish and allowed to solidify. By this approach, the solid agar medium contained approximately 4 x 10⁶ CFU/ml of the bacteria.

A 0.5 mg sample of Ambersorb CB or Ambersorb C solid powder or a 10-ul sample of the slurry form was placed in the center of the inoculated agar plates. As a positive control, 10 *u*l of the 5.2% solution of AgNO₃ was placed in the center of the agar plate.

The treated and control plates were incubated at 30°C for 24 hours. At the end of the incubation period, the diameter (in cm) of the inhibition zone around the chemical treatments was measured.

The results showed that Ambersorb CB with Ag, in slurry and in solid form, effectively inhibited the growth of the bacteria. The solid resin contained approximately 99 ug of Ag and inhibited bacterial growth at a 7.1 cm in diameter zone. The slurry contained approximately 330 ug Ag and inhibited an area of 1.7 cm in diameter of bacterial growth.

AgNO₃ alone demonstrated excellent activity versus the *Bacillus* bacterium. It demonstrated a larger zone (3.7 cm in diameter) than the Ambersorb CB slurry at an equal Ag basis. This is likely due to greater water solubility of the free AgNO₃ which provides for greater diffusion in the agar (larger zone). The solid showed the largest zone at the lowest Ag, but this may be due to the tendency of the solid powder to spread over a larger area than the liquids during the placement on the agar.

The Zone of Inhibition test results are shown below in Table 1 :

**TABLE 1**

| **Samples** | **Phase** | **Sample Size** | **ZOI (CM)** | **Ag (ug) In sample** |
|---|---|---|---|---|
| Ambersorb CB | Slurry | 10 ul | 1.7 | 330 |
| | Solid | 500 ug | 7.1 | 99 |
| Ambersorb C (no Ag) | Slurry | 10 ul | 0 | 0 |
| | Solid | 500 ug | 0 | 0 |
| AgNO₃ | Slurry | 10 ul | 3.7 | 330 |

**Hard Surface Test** (2 minutes contact time) - This test measures the biocides ability to eradicate the bacterial dry film on a hard surface)(CFU/ml = colony forming unit per milliliter)

### Hard Surface 2 Minutes Contact Kill Test

This test is designed to determine the bactericidal effect of the resin treatments on bacteria which are dried onto a solid surface. The contact time was limited to 2 minutes and viable bacteria were recovered in standard growth media.

Sterile glass slides were inoculated with 10 *u*l of a bacterial suspension in pH 7 phosphate buffer (containing approximately 5 x 10⁷ cfu/ml of bacteria) and allowed to dry at 30°C for 45 minutes. The bacterial inoculum formed a dry film on the glass slide surface. By this approach, each glass slide received approximately 5x10⁵ cfu of bacteria per square inch of glass surface.

With Ambersorb CB or Ambersorb C, 1 g of the solid powder or 1 ml of the slurry was placed on the inoculated area of the glass slide. The powder was spread over the surface and was stirred (without disturbing the bacterial film) during the testing period. After 2 minutes of contact time the powder or the slurry was shaken off and the slides were placed in 20 ml of pH 7 buffer solution. This solution was then serial diluted and plated to determine the number of surviving bacteria.

With AgNO₃, 1 ml of the 5.2% solution was used to spread onto the inoculated area of the glass slide. The test was performed the same as described above. The results showed that the slurry and solid forms of the Ambersorb CB resin demonstrated excellent bactericidal activity on the hard surfaces after only 2 minutes contact time. No viable counts were recovered with the slurry form and greater than 99.98% kill was achieved with the solid form.

AgNO₃ alone also showed excellent efficacy against the bacteria, as expected, with no viable counts were recovered. Ambersorb C resin, with no silver component, demonstrated only a minimal (1-log) reduction versus the untreated control.

The results of the hard surface contact test are shown below in Table 2:

**TABLE 2**

| **Samples** | **Phase** | **CFU/ml Bacteria Recovered** | **Ag (mg In sample** |
|---|---|---|---|
| Ambersorb CB | Slurry | <20 | 33 |
| | Solid | 80 | 198 |
| Ambersorb C (no Ag) | Slurry | 5.0E+04 | 0 |
| | Solid | 4.6E+04 | 0 |
| AgNO₃ | Liquid | <20 | 33 |
| 0 | | 5.8E+05 | 0 |

**Speed of Kill** - This test measures the ability of Ambersorb CB to eradicate (cidal effect) the bacteria in an aqueous solution after 2 minutes contact time.

This test is a liquid suspension test in which the bacteria and treated resins are in contact with each other for two minutes in a completely aqueous medium. The extent of kill is determined by measuring survivors in standard growth media.

For this test, one ml of the buffered suspension of *Bacillus subtilis* was further diluted into 9 ml of pH 7 phosphate buffer to yield a final cell concentration of 5x10⁶ cfu/ml.

Following the preparation of the final bacterial test solution, 1 g of Ambersorb CB or Ambersorb C or 1 ml of 31% AgNO₃ was added to the 10 ml sample. The solution was shaken vigorously to mix. After 2 minutes contact time, the solution was serially diluted and plated to determine the number of bacterial survivors.

The results showed that Ambersorb CB resin demonstrated excellent anti-bacterial activity after 2 minutes contact time in solution. No viable *Bacillus* cells were recovered from the treated samples (with > 5 log of kill).

AgNO₃ also demonstrated the same level of antibacterial activity, with no survivors recovered after 2 minutes. Ambersorb C resin, with no silver, provided no activity against the bacteria.

The results of the Speed of Kill Test are shown in Table 3 below:

**Table 3**

| **Samples** | **CFU/ml Bacteria Recovered** | **Ag (mg) in sample** |
|---|---|---|
| Ambersorb CB | 0 | 198 |
| Ambersorb C (no Ag) | 1.8E+05 | 0 |
| AgNO₃ | 0 | 198 |
| 0 | 2.9E+05 | 0 |

### Example 2

### Ambersorb Without Nitric Acid Treatment

10 grams of a carbonaceous adsorbent having a surface area of 750 sq m/g was added to 32.7 grams of 1N AgNO₃ and heated at 80C until just wet. It was then placed in a 110C oven and dried overnight. The spherical beads were then ground to a fine powder for anti-microbial testing. The material was found to contain 5.51% silver by inductively coupled plasma emission.

Testing was conducted as in Example 1 with the following results:

| Zone of Inhibition: | | | | |
|---|---|---|---|---|
| **Samples** | **Phase** | **Sample Size** | **ZOI (CM)** | **Ag (ug) In sample** |
| Ambersorb CB | slurry | 10 ul | 1.1 | 92.0 |
| | Solid | 500 ug | 1.5 | 22.0 |

| Hard Surface: | | | | | |
|---|---|---|---|---|---|
| **Samples** | **Phase** | **Sample Size** | **CFU Bacteria Recovered on 1 in**^{**2**} **slide** | **Log Reduction vs Control** | **Ag (ug) In sample** |
| Ambersorb CB | slurry | 0.76 g | <20 | 4.9 | 7.0 |
| 0 | | | 1.7E+06 | | |

| Speed of Kill (Two minute Contact Time): | | | |
|---|---|---|---|
| **Samples** | **CFU/ml Bacteria Recovered** | **Log Reduction vs Control** | **Ag (mg) In 5.5 ml sample** |
| Ambersorb CB | <10 | 5.3 | 27.6 |
| 0 | 1.9E+06 | | 0 |

### Example 3

### Activated Alumina

3 grams of a powdered activated alumina was added to a solution in which 1.417g crystalline AgNO₃ had been dissolved in 13.4 ml DI water. After stirring and heating at 80C to reduce the quantity of water present the was placed in a 110C oven and dried overnight. The material was found to contain 12.51% silver determined by inductively coupled plasma emission.

Testing was conducted as in Example 1 with the following results:

| Zone of Inhibition: | | | | |
|---|---|---|---|---|
| **Samples** | **Phase** | **Sample Size** | **ZOI (CM)** | **Ag (ug) In sample** |
| Alumina w/ Ag | slurry | 10 ul | 1.2 | 208.8 |
| | Solid | 400 ug | 3.1 | 50 |
| Alumina | slurry | 10 ul | 0 | 0 |
| | Solid | 400 ug | 0 | 0 |

| Hard Surface: | | | | | |
|---|---|---|---|---|---|
| **Samples** | **Phase** | **Sample Size** | **CFU Bacteria Recovered on 1 in**^{**2**} **slide** | **Log Reduction vs Control** | **Ag (ug) In sample** |
| Alumina w/ Ag | slurry | 0.81 g | <20 | 4.9 | 16.9 |
| 0 | | | 1.7E+06 | | |

| Speed of Kill (Two minute Contact Time): | | | |
|---|---|---|---|
| **Samples** | **CFU/ml Bacteria Recovered** | **Log Reduction vs Control** | **Ag (mg) In 5.5 ml sample** |
| Alumina w/ Ag | <10 | 5.3 | 62.5 |
| Alumina | 1.5E+06 | 0.1 | 0 |
| Untreated Control | 1.9E+06 | | 0 |

### Example 4

### Amberchrom CG 300

Amberchrom CG300M polymeric adsorbent in ethanol/ water was washed with DI water and then drained in a buchner funnel to remove surface water. 5 grams of the wetted resin was added to a solution in which 1.52g crystalline AgNO₃ had been dissolved in 20 ml DI water. After stirring and heating at 80C to reduce the quantity of water present the was placed in a 110C oven and dried overnight. The material was kept in its bead form (not ground). The dried material was found to contain 49.15% silver determined by inductively coupled plasma emission. The water wetted adsorbent was found to contain 21.1% solids determined by loss of weight upon drying at 110C overnight.

Testing was conducted as in Example 1 with the following results:

| Zone of Inhibition: | | | | |
|---|---|---|---|---|
| **Samples** | **Phase** | **Sample Size** | **ZOI (CM)** | **Ag (ug) In sample** |
| CG300 w/ Ag | slurry | 10 ul | 1.1 | 820.8 |
| | Solid | 400 ug | 1.5 | 196.6 |
| CG300 | slurry | 10 ul | 0 | 0 |
| | Solid | 400 ug | 0 | 0 |

| Hard Surface: | | | | | |
|---|---|---|---|---|---|
| **Samples** | **Phase** | **Sample Size** | **CFU Bacteria Recovered on 1 in**^{**2**} **slide** | **Log Reduction vs Control** | **Ag (ug) In sample** |
| CG300 w/ Ag | slurry | 0.93 g | <20 | 4.9 | 76.3 |
| CG300 | slurry | 0.97 | 1.3E+05 | 1.1 | 0 |
| 0 | | | 1.7E+06 | | |

| Speed of Kill (Two minute Contact Time): | | | |
|---|---|---|---|
| **Samples** | **CFU/ml Bacteria Recovered** | **Log Reduction vs Control** | **Ag (mg) In 5.5 ml sample** |
| CG300 w/ Ag | <10 | 5.3 | 245.8 |
| CG300 | 1.3E+06 | 0.2 | 0 |
| Untreated Control | 1.9E+06 | | 0 |

## Claims

1. A method for preventing and treating the biological and chemical contamination of a mammalian subject comprising topically administering to said subject a silver impregnated adsorbent.

2. A method for preventing and treating the biological and chemical contamination of a gas stream or mist comprising contacting said gas stream or mist with a silver impregnated adsorbent.

3. A method for preventing biological and chemical contamination of a mammalian subject comprising wearing protective clothing containing a silver impregnated adsorbent.

4. A method for preventing and treating the biological and chemical contamination of a surface comprising topically administering to said surface a silver impregnated adsorbent.

5. A method for preventing and treating the biological and chemical contamination of a mammalian subject comprising topically administering to said subject a silver impregnated carbonaceous adsorbent , wherein further, said silver impregnated carbonaceous adsorbent is co-administered with a material selected from the group consisting of cationic ion exchange resins, anionic ion exchange resins or mixtures of both.
